# EUROPEAN PATENT APPLICATION

(11) **EP 2 958 038 A1**
(43) Date of publication of application: **23.12.2015**
(21) Application number: 14752140.5
(22) Date of filing: 14.02.2014
(51) Int. Cl.: G06F 19/22, C12N 15/115

(54) **SELECTION DEVICE FOR CANDIDATE SEQUENCE INFORMATION FOR SIMILARITY DETERMINATION, SELECTION METHOD, AND USE FOR SUCH DEVICE AND METHOD**

(30) Priority: 15.02.2013 JP 2013027851
(71) Applicant: NEC Solution Innovators, Ltd., Tokyo 136-8627 (JP)
(72) Inventor: AKITOMI Jou, Tokyo 136-8627 (JP); HORII Katsunori, Tokyo 136-8627 (JP)
(74) Representative: Betten & Resch
(86) International application number: PCT/JP2014/053516
(87) International publication number: WO 2014/126213

(57) **Abstract**

The present invention provides a device for determining the similarities between sequence information pieces easily. The candidate selection device 10 of the present invention includes an input unit 11, a sequence storage section 121, a similarity degree storage section 122, a candidate sequence storage section 123, a similarity degree calculation unit 131, a candidate sequence selection unit 132, and an output unit 14. The input unit 11 is used to input information on a sequence group and a virtual sequence group. The similarity degree calculation unit 131 selects a comparison source and a comparison target from the sequence group, and calculates the difference in the frequency of each virtual sequence between the comparison source sequence and the comparison target sequence, as the similarity degree of the comparison target sequence with respect to the comparison source sequence. When the similarity degree of the comparison target sequence with respect to the comparison source sequence satisfies the allowable similarity degree condition set for the virtual sequence group, the candidate sequence selection unit 132 selects the comparison source sequence and the comparison target sequence as a candidate sequence group for determination of similarity between the sequences. By determining the similarities between sequences in the candidate sequence group, a certain sequence and a sequence(s) similar thereto can be selected as a similar sequence information group.

## Description

### Technical Field

The present invention relates to determination of similarity between pieces of sequence information (hereinafter also referred to as "sequence information pieces") in a sequence information group. More specifically, the present invention relates to: a candidate selection method for selecting, from sequence information, candidate sequence information for determination of similarity; a similar information selection method for selecting a similar sequence information group from candidate sequence information; a determination method for determining enrichment of a desired similar sequence information group; and respective devices, programs, and recording media for carrying out these methods.

### Background Art

In recent years, as target-binding molecules that can be substitutes for antibodies, nucleic acid molecules called "aptamers" are being developed. The aptamers generally are prepared by a SELEX (Systematic Evolution of Ligands by Exponential enrichment) method (Patent Document 1, Non-Patent Document 1). In the SELEX method, a plurality of rounds of selection process are performed, each of which includes the step of bringing a target into contact with a nucleic acid library and the step of amplifying nucleic acids bound to the target. Through these selection processes, nucleic acid sequences that bind to the target are enriched from an initial library as the round proceeds. Then, for example, by selecting a plurality of relatively highly enriched nucleic acid sequences in a library as an aptamer candidate group and further evaluating the binding force or the like of the nucleic acid sequences with the target, an aptamer that binds to the target can be determined eventually.

As described above, the aptamer candidate group can be selected on the basis of the degree of enrichment in a library. Thus, in the SELEX method, it is necessary to evaluate the degree of enrichment. Generally, the degree of enrichment is evaluated in the following manner. First, nucleic acid sequences contained in a library in each round are decoded with a sequencer. Then, the number of appearances (hereinafter also referred to as "multiplicity") of the same nucleic acid sequence in the library is counted. On the basis of increase or decrease of this counted number, the degree of enrichment of each nucleic acid sequence is evaluated. For example, the multiplicity mₙ of a nucleic acid sequence X in the n-th round (Rₙ) is compared with the multiplicity mₙ₊₁ of the nucleic acid sequence X in a subsequent round, i.e., the (n+1)-th round (Rₙ₊₁). If the multiplicity mₙ < the multiplicity mₙ₊₁ is satisfied, it can be determined that the nucleic acid sequence X in the round (n+1) is enriched more highly than in the round (n). Also, by comparing the multiplicity mx of the nucleic acid sequence X with the multiplicity m_{Y} of a nucleic acid sequence Y in a library in the same round, it can be determined that the nucleic acid sequence exhibiting a higher multiplicity is enriched more highly than the other.

### Citation List

### Patent Document(s)

Patent Document 1: Japanese Patent No. 2763958

### Non-Patent Document(s)

Non-Patent Document 1: Science. (1990) 249, pp. 505 to 510.

### Summary of the Invention

### Problems to be Solved by the Invention

However, even if an aptamer candidate group is selected on the basis of the degree of enrichment, evaluating the binding force of each one of different nucleic acid sequences with the target requires too much labor and thus is not practical.

On the other hand, a library contains nucleic acid sequences having exactly the same base sequence as a certain nucleic acid sequence (hereinafter also referred to as an "original sequence"), but also may contain similar nucleic acid sequences having a few mismatch bases with respect to the original sequence (hereinafter also referred to as "similar sequences"). The inventors of the present invention found out that the similar sequences may bind to the target with binding forces different from that of the original sequence, for example, but the similar sequences often exhibit the same properties etc. to the target as the original sequence. On this account, the efficiency of aptamer evaluation can be improved by sorting nucleic acid sequences regarded as similar to each other within an allowable range to the same sequence group, rather than sorting exactly the same nucleic acid sequences to the same sequence group. In this case, however, it also takes labor, cost, and time to check the similarities between the plurality of nucleic acid sequences on a one-by-one basis. In particular, in the case where, for example, a large amount of nucleic acid sequence information is obtained with the use of a next-generation sequencer or the like, the cost required for calculation would be very high. Such problems are not specific to nucleic acid sequences, but are common to any sequence information including aligned components.

With the foregoing in mind, it is an object of the present invention to provide devices, methods, programs, and recording media for determining the similarities between sequence information pieces easily.

### Means for Solving Problem

In order to achieve the above object, the present invention provides a candidate selection device for selecting, from a sequence information group including sequence information pieces, a candidate sequence information group including candidate sequence information pieces that serve as candidates for determination of similarity between the sequence information pieces. The candidate selection device includes the following units (a), (b), (c), and (d):
(a) a unit that performs the step of counting the frequency of each virtual sequence information piece included in a virtual sequence information group in each sequence information piece in the sequence information group;
(b) a unit that performs the step of selecting, from the sequence information group, a sequence information piece that serves as a comparison source and a sequence information piece that serves as a comparison target;
(c) a unit that performs the step of calculating the difference between the frequency of each virtual sequence information piece in the comparison source sequence information piece and the frequency of each virtual sequence information piece in the comparison target sequence information piece as the similarity degree of the comparison target sequence information piece with respect to the comparison source sequence information piece; and
(d) a unit that performs the step of selecting, when the similarity degree of the comparison target sequence information piece with respect to the comparison source sequence information piece satisfies an allowable similarity degree condition set for the virtual sequence information group, the comparison source sequence information piece and the comparison target sequence information piece as the candidate sequence information group for determination of similarity between the sequence information pieces.

The present invention also provides a similar information selection device for selecting, from a sequence information group including sequence information pieces, a similar sequence information group including similar sequence information pieces that are similar to each other. The similar information selection device includes the following units (A) and (B):
(A) a unit that performs the step of selecting, from the sequence information group, a candidate sequence information group including candidate sequence information pieces that serve as candidates for determination of similarity between the sequence information pieces; and
(B) a unit that performs the step of contrasting the respective candidate sequence information pieces in the candidate sequence information group with each other and selecting the same and similar sequence information pieces as a similar sequence information group (G3). In similar information selection device, the unit (A) is the candidate selection device according to the present invention.

The present invention also provides a determination device for determining enrichment of a desired similar sequence information group, including the following units (X) and (Y):
(X) a unit that performs the step of selecting, from a sequence information group including sequence information pieces, a desired sequence information piece and a sequence information piece similar thereto as a desired similar sequence information group; and
(Y) a unit that performs the step of determining enrichment of the similar sequence information group from the sum of the multiplicities of the desired sequence information piece and the sequence information piece similar thereto in the similar sequence information group. In the determination device, the unit (X) is the similar information selection device according to the present invention.

The present invention also provides a candidate selection method for selecting, from a sequence information group including sequence information pieces, a candidate sequence information group including candidate sequence information pieces that serve as candidates for determination of similarity between the sequence information pieces. The candidate selection method includes the following steps (a), (b), (c), and (d):
(a) the step of counting the frequency of each virtual sequence information piece included in a virtual sequence information group in each sequence information piece in the sequence information group;
(b) the step of selecting, from the sequence information group, a sequence information piece that serves as a comparison source and a sequence information piece that serves as a comparison target;
(c) the step of calculating the difference between the frequency of each virtual sequence information piece in the comparison source sequence information piece and the frequency of each virtual sequence information piece in the comparison target sequence information piece as the similarity degree of the comparison target sequence information piece with respect to the comparison source sequence information piece; and
(d) the step of selecting, when the similarity degree of the comparison target sequence information piece with respect to the comparison source sequence information piece satisfies an allowable similarity degree condition set for the virtual sequence information group, the comparison source sequence information piece and the comparison target sequence information piece as the candidate sequence information group for determination of similarity between the sequence information pieces.

The present invention also provides a similar information selection method for selecting, from a sequence information group including sequence information pieces, a similar sequence information group including similar sequence information pieces that are similar to each other. The similar information selection method includes the following steps (A) and (B):
(A) the step of selecting, from the sequence information group, a candidate sequence information group including candidate sequence information pieces that serve as candidates for determination of similarity between the sequence information pieces; and
(B) the step of contrasting the respective candidate sequence information pieces in the candidate sequence information group with each other and selecting the same and similar sequence information pieces as a similar sequence information group (G3). In similar information selection method, the step (A) includes the candidate selection method according to the present invention.

The present invention also provides a determination method for determining enrichment of a desired similar sequence information group, including the following steps (X) and (Y):
(X) the step of selecting, from a sequence information group including sequence information pieces, a desired sequence information piece and a sequence information piece similar thereto as a desired similar sequence information group; and
(Y) the step of determining enrichment of the similar sequence information group from the sum of the multiplicities of the desired sequence information piece and the sequence information piece similar thereto in the similar sequence information group. In the determination device, the step (X) includes the similar information selection method according to the present invention.

The present invention also provides a program that can execute on a computer at least one selected from the group consisting of the candidate selection method according to the present invention, the similar information selection method according to the present invention, and the determination method according to the present invention.

The present invention also provides a recording medium having recorded thereon the program according to the present invention.

### Effects of the Invention

According to the present invention, in order to determine the similarities between sequence information pieces, first, a candidate sequence group for determination of similarity is selected. Thus, for example, unlike conventional methods in which the similarities between all the sequence information pieces are checked, the determination of similarity can be carried out easily and efficiently. Thus, the present invention also can reduce labor, time, and cost for determination of the enrichment of aptamers etc., for example.

### Brief Description of Drawings

[FIG. 1] FIG. 1 is a block diagram showing an embodiment of the candidate selection device of the present invention.
[FIG. 2] FIG. 2 is a flowchart illustrating an embodiment of the candidate selection method and the candidate selection program of the present invention.
[FIG. 3] FIG. 3 is a flowchart illustrating the embodiment of the candidate selection method and the candidate selection program of the present invention.
[FIG. 4] FIG. 4 is a block diagram showing an embodiment of the similar information selection device of the present invention.
[FIG. 5] FIG. 5 is a flowchart illustrating an embodiment of the similar information selection method and the similar information selection program of the present invention.
[FIG. 6] FIG. 6 is a flowchart illustrating the embodiment of the similar information selection method and the similar information selection program of the present invention.
[FIG. 7] FIG. 7 is a block diagram showing another embodiment of the similar information selection device of the present invention.
[FIG. 8] FIG. 8 is a flowchart illustrating another embodiment of the similar information selection method and the similar information selection program of the present invention.
[FIG. 9] FIG. 9 is a flowchart illustrating said other embodiment of the similar information selection method and the similar information selection program of the present invention.

### Mode for Carrying out the Invention

In the present invention, the term "sequence information group" means a group including a plurality of sequence information pieces. The plurality of sequence information pieces all may be different from each other, or may include both the same sequence information pieces and different sequence information pieces, for example. The present invention aims to select, in order to determine the similarities between different sequence information pieces, candidate sequence information pieces that serve as candidates for the determination of similarity. Thus, it is preferable that the plurality of sequence information pieces are all different from each other, for example. The number of sequence information pieces included in the sequence information group is not particularly limited.

In the present invention, the term "sequence information" is not particularly limited, and may refer to any information on alignment of components. The component may be, for example, at least one of a character and a symbol, and specific examples thereof include a character or a symbol for indicating the kind of a nucleic acid and a character or a symbol for indicating the kind of an amino acid. Examples of the character or symbol for indicating the kind of a nucleic acid include characters or symbols indicating the kinds of bases, such as A, G, C, T, and U. Examples of the character or symbol indicating the kind of an amino acid include characters or symbols written with three letters such as "Met" and a character or a symbol written with one letter such as "M". Specific examples of the sequence information include sequence information on a nucleic acid sequence and sequence information on an amino acid sequence. The length of the sequence information also can be referred to as the number of components constituting the sequence information. The length of the sequence information is not particularly limited, and the number of the components is, for example, 5 to 200, preferably 10 to 150, and more preferably 20 to 120.

In the present invention, the term "virtual sequence information group" means a group including a plurality of virtual sequence information pieces. The virtual sequence information is sequence information that is virtual and includes components (also referred to as "building blocks") constituting the sequence information. The components can be determined depending on the kind of sequence information in the sequence information group. Specifically, the components are the same as those constituting the sequence information in the sequence information group. The virtual sequence information can be referred to as, for example, information in which the components are aligned in any order. The virtual sequence information group can be referred to as a group including a plurality of information pieces in which the components are aligned in any different orders. The length of the virtual sequence information also can be referred to as the number of components constituting the virtual sequence information. The length of the virtual sequence information is not particularly limited, and the number of the components is, for example, 1 to 10, preferably 1 to 7, and more preferably 1 to 4. It is preferable that the virtual sequence information pieces in the virtual sequence information group all have the same length, for example.

In the present invention, sequence information pieces selected from the sequence information group to be compared or contrasted with each other are referred to as a "comparison source sequence information piece" and a "comparison target sequence information piece", respectively. When a sequence information piece is contrasted with a certain sequence information piece, the former sequence information piece also is referred to as a "comparison target", and the latter sequence information piece also is referred to as a "comparison source".

In the present invention, the term "frequency of a virtual sequence information piece" means the frequency with which the virtual sequence information piece appears in sequence information pieces to be examined, and also can be referred to as, for example, components of the frequency vector or the number of appearances. The term "difference in frequency" means the difference in frequency between two or more sequence information pieces, and is, for example, the difference between the frequency of a sequence information piece as a comparison target and the frequency of a sequence information piece as a comparison source.

In the present invention, the term "similarity degree" means the degree of similarity of a comparison target sequence information piece with respect to a comparison source sequence information piece. In the present invention, the term "allowable similarity degree condition" means the condition for similarity degree under which the comparison target sequence information piece can be a candidate for determination of similarity with respect to the comparison source sequence information piece. The allowable similarity degree condition can be set freely, and, for example, can be set on the basis of the allowable number of mismatch components when two sequence information pieces are contrasted with each other. The contrast of two sequence information pieces is, for example, the contrast of alignment of components between the two sequence information pieces. As the allowable similarity degree condition, it is possible to set, for example, a value obtained by multiplying the allowable number (M) of mismatches when two sequence information pieces are contrasted with each other by the length of the virtual sequence information piece (the number N of the components).

In the present invention, the term "multiplicity" means, in a sequence information group including a plurality of sequence information pieces, the number of exactly the same sequence information pieces, and also can be referred to as the number of appearances, for example. In the present invention, the term "similar information multiplicity" means, in a sequence information group including a plurality of sequence information pieces, the sum of the multiplicities of exactly the same sequence information piece and another sequence information piece similar thereto. When there are two or more sequence information pieces similar to the sequence information piece, the sum of the multiplicities of the sequence information piece and each of the other sequence information pieces similar thereto is set to the similar information multiplicity between the sequence information piece and each of the other sequence information pieces similar thereto, for example.

(Candidate selection device and candidate selection method of the present invention)

As described above, the candidate selection device of the present invention is a candidate selection device for selecting, from a sequence information group including sequence information pieces, a candidate sequence information group including candidate sequence information pieces that serve as candidates for determination of similarity between the sequence information pieces. The candidate selection device includes the following units (a), (b), (c), and (d):
(a) a unit that performs the step of counting the frequency of each virtual sequence information piece included in a virtual sequence information group in each sequence information piece in the sequence information group;
(b) a unit that performs the step of selecting, from the sequence information group, a sequence information piece that serves as a comparison source and a sequence information piece that serves as a comparison target;
(c) a unit that performs the step of calculating the difference between the frequency of each virtual sequence information piece in the comparison source sequence information piece and the frequency of each virtual sequence information piece in the comparison target sequence information piece as the similarity degree of the comparison target sequence information piece with respect to the comparison source sequence information piece; and
(d) a unit that performs the step of selecting, when the similarity degree of the comparison target sequence information piece with respect to the comparison source sequence information piece satisfies an allowable similarity degree condition set for the virtual sequence information group, the comparison source sequence information piece and the comparison target sequence information piece as the candidate sequence information group for determination of similarity between the sequence information pieces.

In the candidate selection device of the present invention, it is preferable that the virtual sequence information group includes virtual sequence information pieces constituted by the same components as components constituting the sequence information pieces.

In the candidate selection device of the present invention, it is preferable that the unit (c) is a unit that performs the following steps (c1) and (c2):
(c1) the step of determining, regarding each of the virtual sequence information pieces, the difference between the frequency thereof in the comparison source sequence information piece and the frequency thereof in the comparison target sequence information piece; and
(c2) the step of calculating, as the similarity degree of the comparison target sequence information piece with respect to the comparison source sequence information piece, the absolute value of the sum of positive differences only or the sum of negative differences only among the differences in frequency of the respective virtual sequence information pieces.

In the candidate selection device of the present invention, it is preferable that the allowable similarity degree condition is a condition set based on the allowable number of mismatches when two sequence information pieces are contrasted with each other. Contrast of two sequence information pieces also can be referred to as alignment of two sequence information pieces.

In the candidate selection device of the present invention, it is preferable that, for example, the sequence information pieces are base sequences, and components constituting the sequence information pieces are bases A, G, C, T, and U.

In the candidate selection device of the present invention, it is preferable that the virtual sequence information pieces have a base length of 1- to 10-mer, for example.

In the candidate selection device of the present invention, it is preferable that the virtual sequence information pieces in the virtual sequence information group all have the same base length.

In the candidate selection device of the present invention, it is preferable that the allowable similarity degree condition is a condition set based on the allowable number of mismatch bases when two sequence information pieces are contrasted with each other.

In the candidate selection device of the present invention, it is preferable that the allowable similarity degree condition is a value obtained by multiplying the allowable number (M) of mismatch bases when two sequence information pieces are contrasted with each other by the base length (N) of the virtual sequence information piece.

Preferably, the candidate selection device of the present invention further includes the following unit (e):
(e) a unit that repeats the respective steps performed by the units (b), (c), and (d). In this case, the unit (b) preferably selects, every time the steps are performed, a different sequence information piece from the sequence information group as the comparison source sequence information piece, for example.

As described above, the candidate selection method of the present invention is a candidate selection method for selecting, from a sequence information group including sequence information pieces, a candidate sequence information group including candidate sequence information pieces that serve as candidates for determination of similarity between the sequence information pieces, including the candidate selection method includes the following steps (a), (b), (c), and (d). Unless otherwise stated, descriptions regarding the candidate selection device of the present invention also apply to the candidate selection method of the present invention. The steps (a), (b), (c), and (d) are:
(a) the step of counting the frequency of each virtual sequence information piece included in a virtual sequence information group in each sequence information piece in the sequence information group;
(b) the step of selecting, from the sequence information group, a sequence information piece that serves as a comparison source and a sequence information piece that serves as a comparison target;
(c) the step of calculating the difference between the frequency of each virtual sequence information piece in the comparison source sequence information piece and the frequency of each virtual sequence information piece in the comparison target sequence information piece as the similarity degree of the comparison target sequence information piece with respect to the comparison source sequence information piece; and
(d) the step of selecting, when the similarity degree of the comparison target sequence information piece with respect to the comparison source sequence information piece satisfies an allowable similarity degree condition set for the virtual sequence information group, the comparison source sequence information piece and the comparison target sequence information piece as the candidate sequence information group for determination of similarity between the sequence information pieces.

In the candidate selection method of the present invention, it is preferable that the virtual sequence information group includes virtual sequence information pieces constituted by the same components as components constituting the sequence information pieces.

In the candidate selection method of the present invention, it is preferable that the step (c) includes the following steps (c1) and (c2):
(c1) the step of determining, regarding each of the virtual sequence information pieces, the difference between the frequency thereof in the comparison source sequence information piece and the frequency thereof in the comparison target sequence information piece; and
(c2) the step of calculating, as the similarity degree of the comparison target sequence information piece with respect to the comparison source sequence information piece, the absolute value of the sum of positive differences only or the sum of negative differences only among the differences in frequency of the respective virtual sequence information pieces.

In the candidate selection method of the present invention, it is preferable that the allowable similarity degree condition is a condition set based on the allowable number of mismatches when two sequence information pieces are contrasted with each other.

In the candidate selection method of the present invention, it is preferable that the sequence information pieces are base sequences, and components constituting the sequence information pieces are bases A, G, C, T, and U.

In the candidate selection method of the present invention, it is preferable that the virtual sequence information pieces have a base length of 1- to 10-mer.

In the candidate selection method of the present invention, it is preferable that the virtual sequence information pieces in the virtual sequence information group all have the same base length.

In the candidate selection method of the present invention, it is preferable that the allowable similarity degree condition is a condition set based on the allowable number of mismatch bases when two sequence information pieces are contrasted with each other.

In the candidate selection method of the present invention, it is preferable that the allowable similarity degree condition is a value obtained by multiplying the allowable number (M) of mismatch bases when two sequence information pieces are contrasted with each other by the base length (N) of the virtual sequence information piece.

Preferably, the candidate selection method of the present invention further includes the following step (e). In this case, the step (b) preferably is such that, every time the steps are performed, a different sequence information piece is selected from the sequence information group as the comparison source sequence information piece. The step (e) is:
(e) the step of repeating the steps (b), (c), and (d).

In the candidate selection method of the present invention, it is preferable that the respective steps are all executed on a computer. In the candidate selection method of the present invention, the respective steps all may be executed by the candidate selection device of the present invention, for example.

A specific embodiment of the present invention will be described with reference to the accompanying drawings. It is to be noted, however, that the present invention is by no means limited by the following embodiment. Hereinafter, "sequence information" is referred to as a "sequence", and a "sequence information group" is referred to as a sequence group".

### [Embodiment 1]

Embodiment 1 relates to the candidate selection device and the candidate selection method of the present invention. The present embodiment is directed to an example where the sequence is a base sequence of a nucleic acid.

According the present embodiment, from a base sequence group including a plurality of base sequences, a candidate sequence group including candidate sequences that serve as candidates for determination of similarity between the base sequences can be selected.

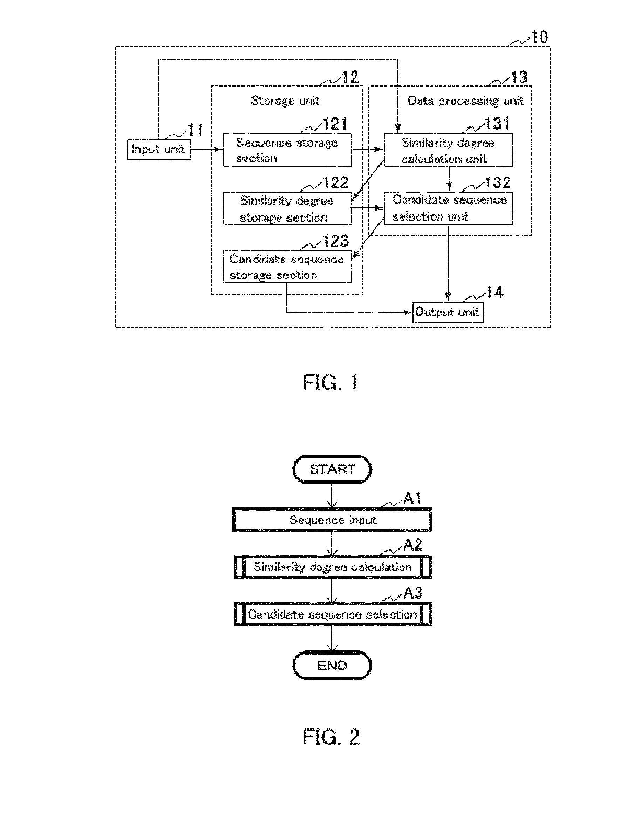

FIG. 1 shows an example of the configuration of the candidate selection device of the present embodiment. As shown in FIG. 1, the candidate selection device10 includes: an input unit 11; a sequence storage section 121, a similarity degree storage section 122, and a candidate sequence storage section 123; a similarity degree calculation unit 131 and a candidate sequence selection unit 132; and an output unit 14. The similarity degree calculation unit 131 and the candidate sequence selection unit 132 may be incorporated in a data processing unit (data processing device) 13, which is hardware, as shown in FIG. 1, for example, or alternatively, they may be software or hardware with the software installed therein. The respective storage sections 121, 122, and 123 may be incorporated in the storage unit 12, which is hardware, as shown in FIG. 1, for example. The data processing unit 13 may include a CPU and the like.

The sequence storage section 121 is connected electrically to the input unit 11 and the similarity degree calculation unit 131. The similarity degree storage section 122 is connected electrically to the similarity degree calculation unit 131 and the candidate sequence selection unit 132. The candidate sequence storage section 123 is connected electrically to the candidate sequence selection unit 132 and the output unit 14. The input unit 11 may be connected electrically to the similarity degree calculation unit 131. The similarity degree calculation unit 131 may be connected electrically to the candidate sequence selection unit 132. The candidate sequence selection unit 132 may be connected electrically to the output unit 14. The candidate selection device10 may perform data processing, for example, by storing information in the storage unit 12 and then outputting the stored information to the data processing unit 13, or by inputting the information to the data processing unit 13.

The input unit 11 is a unit for inputting information on a sequence group and a virtual sequence group (an input device). The input unit 11 is not particularly limited, and examples thereof include: ordinary input units provided in a computer, such as a keyboard and a mouse; input files; and other computers. The input unit 11 may be, for example, a unit for reading out information on the sequence group and the virtual sequence group stored in a database. In this case, for example, sequence information stored in a server in advance is read out by the input unit 11 through a line network. The input unit 11 may include a communication interface, for example.

The number of sequences to be inputted in the sequence group is not particularly limited, and the lower limit is, for example, 5, preferably 10, and the upper limit is, for example, 10,000,000, preferably 1,000,000. The sequence information item to be inputted is, for example, the order in which components constituting the sequence are aligned, i.e., the alignment of bases. The length of the sequence is not particularly limited, and is, for example, 5- to 200-mer, preferably 10- to 150-mer, and more preferably 20- to 120-mer.

The number of virtual sequences in the virtual sequence group is not particularly limited, and can be determined as appropriate depending on the base length(s) of the virtual sequences. The lower limit of the base length is, for example, 1-mer, preferably 2-mer, and more preferably 3-mer, and the upper limit of the base length is, for example, 10-mer, preferably 9-mer, more preferably 8-mer, and still more preferably 7-mer. Preferably, the respective virtual sequences in the virtual sequence group all have the same length.

In the case where the components constituting the virtual sequences are four bases (A, C, G, and T or U) and the base length of the virtual sequences is n (a positive number), the number of the virtual sequences in the virtual sequence group is, for example, 4 to the n-th power (4ⁿ). Specific examples are as follows: when the components are four bases A, C, G, and T, the number of 1-mer virtual sequences is 4¹, i.e., 4 (A, C, G, and T), and the number of 2-mer virtual sequences is 4², i.e., 16 (AA, AC, AG, AT, CC, CA, CG, CT, GG, GA, GC, GT, TT, TA, TC, and TG).

The similarity degree calculation unit 131 performs: as the step (a), the step of counting, regarding each sequence in the sequence group, the frequency of each virtual sequence in the virtual sequence group; as the step (b), the step of selecting a comparison source sequence and a comparison target sequence from the sequence group; and as the step (c), the step of calculating the similarity degree of the comparison target sequence with respect to the comparison source sequence. The order of the steps (a), (b), and (c) is not particularly limited, and they may be in random order.

The calculation of the similarity degree in the step (c) can be performed in the following manner, as described above: as the step (c1), regarding each virtual sequence, the difference (Sₙ - Tₙ) between the frequency (Sₙ) thereof in the comparison source sequence and the frequency (Tₙ) thereof in the comparison target sequence is determined, and as the step (c2), the absolute value of the sum of positive differences only or the sum of negative differences only among the thus-determined differences (Sₙ - Tₙ) in frequency is determined. That is, the absolute value of the sum is set to the similarity degree.

The candidate sequence selection unit 132 selects candidate sequences for determination of similarity between sequences, on the basis of the similarity degree of the comparison target sequence with respect to the comparison source sequence and the allowable similarity degree condition set for the virtual sequence group. A plurality of candidate sequences selected here forms a candidate sequence group.

The allowable similarity degree condition can be set on the basis of the allowable number of mismatch bases when two sequence information pieces are contrasted with each other. Specific examples of the allowable similarity degree condition include a value (N × M) obtained by multiplying the allowable number (M) of mismatch bases by the base length (N) of the virtual sequence. For example, when the virtual sequence (A, C, G, and T) has a base length of N = 1 and the allowable number of mismatch bases is set to M = 2, the allowable condition (N × M) is 1 × 2 = 2. When the similarity degree is 2 or less, the similarity degree is not more than the numerical value set as the allowable condition and satisfies the allowable condition. Thus, the comparison source sequence and the comparison target sequence are selected as candidate sequences for determination of similarity between sequences. On the other hand, when the similarity degree is more than 2, the similarity degree exceeds the numerical value set as the allowable condition and does not satisfy the allowable condition. Thus, the comparison target sequence is not selected as a candidate sequence for determining the similarity to the comparison source sequence.

The reason why a value (N × M) obtained by multiplying the allowable number (M) of mismatch bases by the base length (N) of the virtual sequence is set as an example of the allowable condition is as follows. For example, when the following two sequences are aligned with each other, one base indicated with a capital letter is a mismatch base. When the frequencies of virtual sequences with a base length of N = 2 are counted in these sequences, the underlined part in the source sequence Seq1 to be examined is counted as cg and gg, whereas the underlined part in the target sequence Seq2 to be examined is counted as cA and Ag. That is, even if the allowable number of mismatch bases is 1, the presence of one mismatch changes the counted number of virtual sequences by two at most. Accordingly, by multiplying the allowable number (M) of mismatch bases by the base length (N) of the virtual sequence, it is possible to correct the influence on counting.
Source sequence Seq1 to be examined: aaccggtt
Target sequence Seq2 to be examined: aaccAgtt

The output unit (output device) 14 is not particularly limited, as long as it is a unit that outputs results obtained from the candidate sequence selection unit 132. Also, the output unit 14 may be a unit that outputs information stored in the candidate sequence storage section 123. The output unit 14 is not particularly limited, and examples thereof include: ordinary output devices provided in a computer, such as a display device and a printer; output files; and other computers.

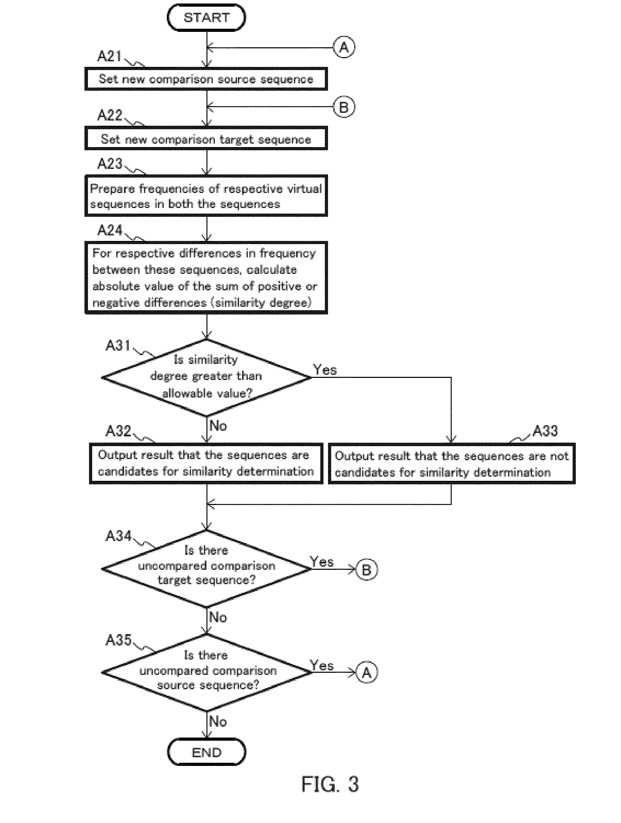

Next, the candidate selection method of the present embodiment will be described with reference to the flowcharts of FIGs. 2 and 3. The candidate selection method of the present embodiment includes the step A1 (sequence input), the step A2 (similarity degree calculation), and the step A3 (candidate sequence selection).

### (A1) Sequence input

Respective sequences in a sequence group and respective virtual sequences in a virtual sequence group are inputted and stored in the sequence storage section 121. An information item on the sequence group and the virtual sequence group may be, for example, the order of bases in a sequence.

### (A2) Similarity degree calculation

From the sequence group, a new comparison source sequence is set (A21) and a new comparison target sequence is set (A22). The frequencies of each virtual sequence in the comparison source sequence and the comparison target sequence set in the above are counted, respectively. Then, regarding each virtual sequence, the difference between the frequency thereof in the comparison source sequence and the frequency thereof in the comparison target sequence is determined, and the sum of positive differences only or the sum of negative differences only is calculated. Specifically, when there are n (n is a positive number) virtual sequences, n frequencies (S₁, ····, Sₙ) are obtained as the frequencies of the respective virtual sequences in the comparison source sequence, and n frequencies (T₁, ····, Tₙ) are obtained as the frequencies of the respective virtual sequences in the comparison target sequence. Then, regarding each of the frequencies of the respective virtual sequences, the difference between the frequency thereof in the comparison source sequence and the frequency thereof in the comparison target sequence, i.e., (S₁ - T₁), ···· , (Sₙ - Tₙ), is determined, and the sum of positive differences only or the sum of negative differences only are calculated, and the absolute value of the sum is determined. The absolute value of the sum is the similarity degree of the comparison target sequence with respect to the comparison source sequence.

### (A3) Candidate sequence selection

Subsequently, whether or not the similarity degree satisfies the allowable value for similarity degree, i.e., whether or not the similarity degree is larger than the allowable value is determined (A31). When the flow goes to NO, i.e., when the similarity degree is smaller than the allowable value, it is determined that the comparison target sequence has an allowable number of mismatches with respect to the comparison source sequence, and the result that the comparison source sequence and the comparison target sequence are candidate sequences for determination of similarity is outputted (A32). On the other hand, when the flow goes to YES, i.e., when the similarity degree is greater than the allowable value, it is determined that the comparison target sequence has an unallowable number of mismatch bases with respect to the comparison source sequence, and the result that the comparison target sequence is not a candidate sequence for determination of similarity is outputted (A33).

Thereafter, whether or not there is a comparison target sequence that has not yet been compared is checked (A34). When the flow goes to YES, i.e., when there is an uncompared comparison target sequence, the flow goes back to the step A22 and the same steps are performed subsequently. When the flow goes to NO, i.e., when there is no uncompared comparison target sequence, whether or not there is an uncompared comparison source sequence is checked further (A35). When the flow goes to YES, i.e., when there is an uncompared comparison source sequence, the flow goes back to the step A21 and the same steps are performed subsequently. When the flow goes to NO, i.e., when there is no uncompared comparison source sequence, the process is terminated. In the case where a certain sequence set as a comparison source sequence has already been compared with another sequence set as a comparison target sequence, the comparison between the former sequence as the comparison target sequence and the latter sequence as the comparison source sequence may be omitted, and the result of the comparison may be used.

The steps A2 and A3 will be described with reference to a further specific example where the virtual sequences have a base length of 1-mer.

First, assume that the virtual sequences with a base length of N = 1 are the following four kinds, the comparison source sequence is the following Seq3, and the comparison target sequence is the following Seq4. Then, the number of mismatch bases allowable so as to be candidates for determination of similarity when two sequences are aligned is M, and the allowable value is N × M = 1 × M = M.
Virtual sequences: A, C, G and T
Comparison source sequence Seq3: ACGTACGT
Comparison target sequence Seq4: AAGAACAT

The frequencies {fA, fC, fG, fT} of the respective virtual sequences (A, C, G, T) in the comparison source sequence Seq3 and the comparison target sequence Seq4 are as follows: {2, 2, 2, 2} in SEQ1; and {5, 1, 1, 1} in Seq2. The differences in the respective frequencies {fA, fC, fG, fT} are as follows: A (2 - 5 = -3), C (2 - 1 = 1), G (2 - 1 = 1), and T (2 - 1 = 1). The absolute value of the sum of the negative differences (-3 + 0 + 0 + 0 = -3) is 3, and the absolute value of the sum of the positive differences (0 + 1 + 1 + 1 = 3) is 3. This absolute value 3 is the similarity degree of the comparison target sequence Seq4 with respect to the comparison source sequence Seq3, and indicates that the comparison target sequence Seq4 has at least three mismatch bases when it is aligned with the comparison source sequence Seq3. When the upper limit of the allowable number of mismatch bases M is set to 2, for example, the allowable value is N × M = 1 × 2 = 2. Thus, the contrast of the calculated similarity degree with the allowable value reveals that: the similarity degree of 3 > the allowable value of 2, so that the comparison target sequence Seq4 is excluded from candidate sequences for determining the similarity to the comparison source sequence Seq3. On the other hand, when the upper limit of the allowable number of mismatch bases M is set to 3, for example, the allowable value is N × M = 1 × 3 = 3. Thus, the contrast of the calculated similarity degree with the allowable value reveals that: the similarity degree of 3 = the allowable value of 3, so that the comparison target sequence Seq4 is selected as a candidate sequence for determining the similarity to the comparison source sequence Seq3.

As described above, when the comparison target sequence satisfies the allowable condition, the comparison target sequence and the comparison source sequence are selected as candidate sequences for determination of similarity. In other words, the comparison target sequence and the comparison source sequence are selected as a candidate sequence group. On the other hand, when the comparison target sequence does not satisfy the allowable condition, the comparison target sequence is not selected as a candidate sequence for determination of similarity. Also, when the comparison source sequence does not have any comparison target sequence satisfying the allowable condition, the comparison source sequence also is not selected as a candidate sequence for determination of similarity.

In the candidate selection device 10 of the present embodiment, the input unit 11 may be connected electrically to the similarity degree calculation unit 131, and the similarity degree calculation unit 131 may be connected electrically to the candidate sequence selection unit 132. The candidate selection device 10 may include the respective storage sections, or may not include the respective storage sections, for example. In this case, the similarity degree calculation unit 131 may calculate the similarity degree for each sequence inputted by the input unit 11, and the candidate sequence selection unit 132 may select candidate sequences using the thus-calculated similarity degrees, for example.

### (Similar information selection device and similar information selection method of the present invention)

As described above, the similar information selection device of the present invention is a similar information selection device for selecting, from a sequence information group including sequence information pieces, a similar sequence information group including similar sequence information pieces that are similar to each other. The similar information selection device includes the following units (A) and (B):
(A) a unit that performs the step of selecting, from the sequence information group, a candidate sequence information group including candidate sequence information pieces that serve as candidates for determination of similarity between the sequence information pieces; and
(B) a unit that performs the step of contrasting the respective candidate sequence information pieces in the candidate sequence information group with each other and selecting the same and similar sequence information pieces as a similar sequence information group (G3). In the similar information selection device, the unit (A) is the candidate selection device according to the present invention.

In the similar information selection device of the present invention, the unit (A) is not limited as long as it is the candidate selection device of the present invention, and the descriptions as to the candidate selection device of the present invention also apply to the unit (A).

In the similar information selection device of the present invention, the sequence information group preferably is a group including different sequence information pieces selected from a sequence information group (G) including the same sequence information pieces and the different sequence information pieces.

In the similar information selection device of the present invention, it is preferable that the unit (B) is a unit that performs the following steps (B1), (B2), (B3), (B4), and (B5):
(B1) the step of selecting, from the candidate sequence information group, a candidate sequence information piece that serves as a comparison source and a candidate sequence information piece that serves as a comparison target;
(B2) the step of determining whether the comparison target candidate sequence information piece is similar to the comparison source candidate sequence information piece;
(B3) the step of calculating the sum of the multiplicities of the comparison source candidate sequence information piece and the comparison target candidate sequence information piece similar thereto, and setting the calculated sum to the similar information multiplicity of the comparison source candidate sequence information piece;
(B4) the step of selecting, from the candidate sequence information group, a different candidate sequence information piece as a new candidate sequence information piece that serves as a comparison source, and repeating the steps (B1), (B2) and (B3); and
(B5) the step of selecting, among the candidate sequence information pieces, a candidate sequence information piece exhibiting the largest similar information multiplicity and a candidate sequence information piece similar thereto as a similar sequence information group (G3).

In the step (B2), the method for determining whether the comparison target candidate sequence is similar to the comparison source candidate sequence is not particularly limited, and known methods can be used. Specifically, whether or not the comparison target candidate sequence is similar to the comparison source candidate sequence can be determined on the basis of the allowable number of mismatches (different components) when the sequences are aligned with each other. A specific example is as follows, for example: in the case where the number of mismatches when the sequences are aligned with each other is greater than the allowable number of mismatches, it can be determined that they are not similar to each other, whereas, in the case where the number of mismatches is equal to or smaller than the allowable number of mismatches, it can be determined that they are similar to each other. The allowable number of mismatches is not particularly limited, and can be determined freely.

The multiplicity is reset to 0 while the subsequent steps are repeated. Thus, the multiplicity in the step (B3) is initial information on each sequence, so that it also is referred to as an "initial multiplicity". The multiplicity reset to 0 during the subsequent steps also is referred to as the "multiplicity 0" or the "reset multiplicity".

In the similar information selection device of the present invention, it is preferable that the unit (B) is a unit that further performs the following steps (B6), (B7), and (B8). Recalculation of similar information multiplicity means, for example, to reset the already acquired similar information multiplicity and newly calculate a similar information multiplicity. The steps (B6), (B7), and (B8) are:
(B6) the step of resetting, among the candidate sequence information pieces, the multiplicity of the candidate sequence information piece exhibiting the largest similar information multiplicity and the multiplicity of the candidate sequence information piece similar thereto to 0;
(B7) the step of recalculating the similar information multiplicities of other candidate sequence information pieces exhibiting a multiplicity other than 0; and
(B8) the step of reselecting, among the other candidate sequence information pieces, a candidate sequence information piece exhibiting the largest similar information multiplicity and a candidate sequence information piece similar thereto as a similar sequence information group.

In the similar information selection device of the present invention, it is preferable that the unit (B) further performs the following step (B9):
(B9) the step of resetting, among the other candidate sequence information pieces, the multiplicity of the candidate sequence information piece exhibiting the largest similar information multiplicity and the multiplicity of the candidate sequence information piece similar thereto to 0 and repeating the steps (B7) and (B8).

As described above, by repeating the selection of a similar candidate group on the basis of the largest similar information multiplicity and recalculation of the similar information multiplicity, a plurality of similar sequence information groups can be selected. It is preferable to perform reselection of the similar sequence information group until, for example, the multiplicities of all the candidate sequences is reset to 0.

In the similar information selection device of the present invention, it is preferable that the unit (B) excludes, as a combination of the comparison source candidate sequence information piece and the comparison target candidate sequence information piece in the step (B1), a combination that has already been made.

In the similar information selection device of the present invention, examples of an information item on sequence information may include, in addition to the order in which components constituting each sequence is aligned, the multiplicity of each sequence. In this case, it is preferable that the sequences included in the sequence group are all different from each other. Also, in the case where the multiplicity is not included as the information item of sequence information, the similar information selection device of the present invention may include, for example, the following unit (B') that perform the step of counting the multiplicity. In this case, the sequences included in the sequence group may include, for example, in addition to different sequences, sequences in which the order of components is exactly the same. The unit (B') is:
(B') a unit that performs the step of counting, as the multiplicity, the number of exactly the same sequence information pieces in the sequence information group.

As described above, the similar information selection method of the present invention is a similar information selection method for selecting, from a sequence information group including sequence information pieces, a similar sequence information group including similar sequence information pieces that are similar to each other. The similar information selection method includes the following steps (A) and (B):
(A) the step of selecting, from the sequence information group, a candidate sequence information group including candidate sequence information pieces that serve as candidates for determination of similarity between the sequence information pieces; and
(B) the step of contrasting the respective candidate sequence information pieces in the candidate sequence information group with each other and selecting the same and similar sequence information pieces as a similar sequence information group (G3). In the similar information selection method, the step (A) includes the candidate selection method according to the present invention.

In the similar information selection method of the present invention, it is preferable that the step (B) includes the following steps (B1), (B2), (B3), (B4), and (B5):
(B1) the step of selecting, from the candidate sequence information group, a candidate sequence information piece that serves as a comparison source and a candidate sequence information piece that serves as a comparison target;
(B2) the step of determining whether the comparison target candidate sequence information piece is similar to the comparison source candidate sequence information piece;
(B3) the step of calculating the sum of the multiplicities of the comparison source candidate sequence information piece and the comparison target candidate sequence information piece similar thereto, and setting the calculated sum to the similar information multiplicity of the comparison source candidate sequence information piece; (B4) the step of selecting, from the candidate sequence information group, a different candidate sequence information piece as a new candidate sequence information piece that serves as a comparison source, and repeating the steps (B1), (B2) and (B3); and
(B5) the step of selecting, among the candidate sequence information pieces, a candidate sequence information piece exhibiting the largest similar information multiplicity and a candidate sequence information piece similar thereto as a similar sequence information group (G3).

In the similar information selection method of the present invention, it is preferable that the step (B) further includes the following steps (B6), (B7) and (B8):
(B6) the step of resetting, among the candidate sequence information pieces, the multiplicity of the candidate sequence information piece exhibiting the largest similar information multiplicity and the multiplicity of the candidate sequence information piece similar thereto to 0;
(B7) the step of recalculating the similar information multiplicities of other candidate sequence information pieces exhibiting a multiplicity other than 0; and
(B8) the step of reselecting, among the other candidate sequence information pieces, a candidate sequence information piece exhibiting the largest similar information multiplicity and a candidate sequence information piece similar thereto as a similar sequence information group.

In the similar information selection method of the present invention, it is preferable that the step (B) further includes the following step (B9):
(B9) the step of resetting, among the other candidate sequence information pieces, the multiplicity of the candidate sequence information piece exhibiting the largest similar information multiplicity and the multiplicity of the candidate sequence information piece similar thereto to 0 and repeating the steps (B7) and (B8).

In the similar information selection method of the present invention, it is preferable that the step (B) includes excluding, as a combination of the comparison source candidate sequence information piece and the comparison target candidate sequence information piece in the step (B1), a combination that has already been made.

In the similar information selection method of the present invention, it is preferable that the respective steps are all executed on a computer. In the similar information selection method of the present invention, the respective steps all may be executed by the similar information selection device of the present invention, for example.

A more specific embodiment of the present invention will be described below with reference to the accompanying drawings. It is to be noted, however, that the present invention is by no means limited to the following embodiment. In the present embodiment, descriptions in Embodiment 1 also apply to the selection of the candidate sequence group. Hereinafter, "sequence information" is referred to as a "sequence", and a "sequence information group" is referred to as a "sequence group".

### [Embodiment 2]

Embodiment 2 relates to the similar information selection device and the similar information selection method of the present invention. The present embodiment is directed to an example where the sequence is a base sequence of a nucleic acid. Unless otherwise stated, descriptions in Embodiment 1 also apply to the present embodiment.

According the present embodiment, from a base sequence group including a plurality of base sequences, candidate sequences that serve as candidates for determination of similarity between the base sequences are selected, and from a candidate sequence group including the plurality of candidate sequences, similar sequences that are similar to each other are selected as a similar sequence group.

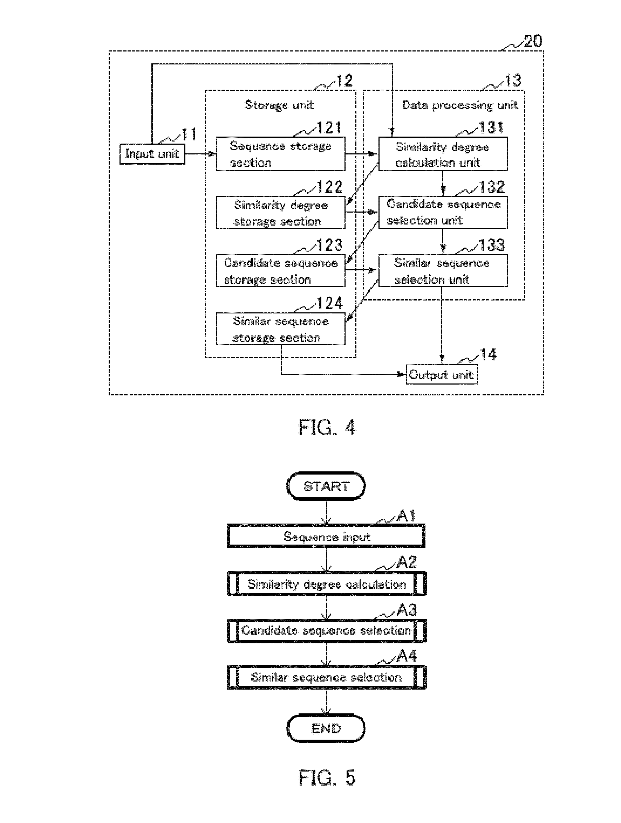

FIG. 4 shows an example of the similar information selection device of the present embodiment. In FIG. 4, components identical to those in the candidate selection device 10 of FIG. 1 are given the same reference numerals. As shown in FIG. 4, the similar information selection device 20 includes: an input unit 11; a sequence storage section 121, a similarity degree storage section 122, a candidate sequence storage section 123, and a similar sequence storage section 124; a similarity degree calculation unit 131, a candidate sequence selection unit 132, and a similar sequence selection unit 133; and an output unit 14. The similarity degree calculation unit 131, the candidate sequence selection unit 132, and the similar sequence selection unit 133 may be incorporated in a data processing unit 13, which is hardware, as shown in FIG. 4, for example, or alternatively, they may be software or hardware with the software installed therein. The storage sections 121, 122, 123, and 124 may be incorporated in the storage unit 12, which is hardware, as shown in FIG. 4, for example. The data processing unit 13 may include a CPU and the like.

The candidate sequence storage section 123 further is connected electrically to the similar sequence selection unit 133. The similar sequence storage section 124 is connected electrically to the similar sequence selection unit 133 and the output unit 14. The candidate sequence selection unit 132 may be connected electrically to the similar sequence selection unit 133. The similar sequence selection unit 133 may be connected electrically to the output unit 14. The similar information selection device 20 may perform data processing, for example, by storing information in the storage unit 12 and then outputting the stored information to the data processing unit 13, or by inputting the information to the data processing unit 13.

In the present embodiment, the sequence information items to be inputted preferably include, in addition to the order in which components constituting each sequence is aligned as described above, the multiplicity of each sequence. In the case where the multiplicity is included as the information item, it is preferable that the sequences included in the sequence group are all different from each other.

In the case where the multiplicity is not included as the information item, the similar information selection device of the present embodiment may further include the above-described unit (B'), for example. The unit (B') can count, as the multiplicity, the number of exactly the same sequences in the sequence group.

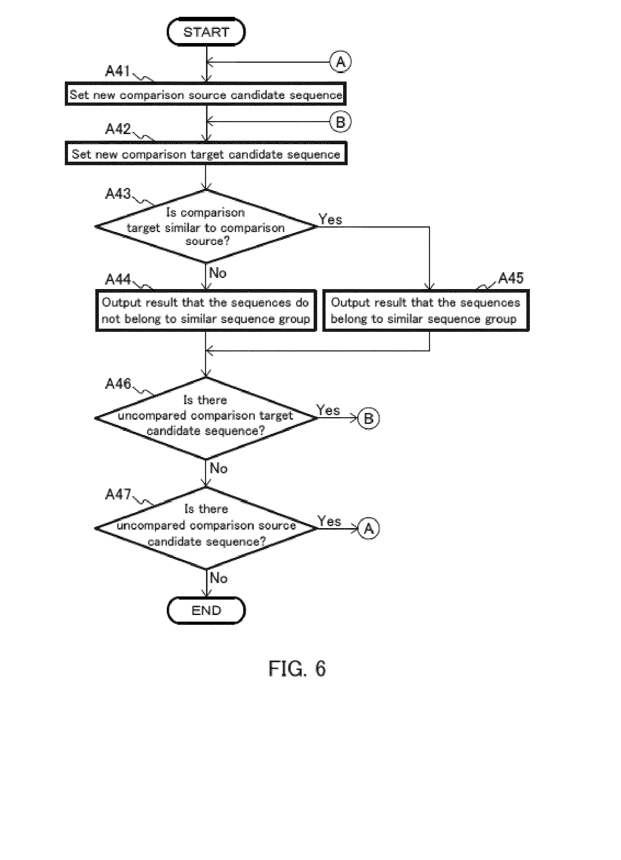

Next, the similar information selection method of the present embodiment will be described with reference to the flowcharts of FIGs. 5 and 6. The similar information selection method of the present embodiment includes the step A1 (sequence input), the step A2 (similarity degree calculation), the step A3 (candidate sequence selection), and the step A4 (similar sequence selection). In FIG. 5, steps identical to those in FIG. 2 are given the same reference numerals.

The steps A1, A2, and A3 can be performed in the same manner as in Embodiment 1. Specifically, the steps A1, A2, and A3 can be performed according to the flowchart of FIG. 3. In the sequence input, examples of the information item on the sequence group include the order in which bases are aligned in each sequence and the multiplicity of each sequence. Examples of the information item on the virtual sequence group include the order in which bases are aligned in each sequence.

### (A4) Similar sequence selection

From the candidate sequence group selected in the step A3, a new comparison source candidate sequence is set (A41) and a new comparison target candidate sequence is set (A42). Then, whether or not the comparison target candidate sequence set in the above is similar to the comparison source candidate sequence is determined (A43). When the flow goes to NO, i.e., when the comparison target candidate sequence is not similar to the comparison source candidate sequence, the result that the comparison target candidate sequence does not belong to a similar sequence group with respect to the comparison source candidate sequence is outputted (A44). On the other hand, when the flow goes to YES, i.e., when the comparison target candidate sequence is similar to the comparison source candidate sequence, the result that the comparison target candidate sequence belongs to a similar sequence group with respect to the comparison source candidate sequence is outputted (A45).

Thereafter, whether or not there is a comparison target candidate sequence that has not yet been compared with the comparison source candidate sequence is checked (A46). When the flow goes to YES, i.e., when there is an uncompared comparison target candidate sequence, the flow goes back to the step A42 and the same steps are performed subsequently. When the flow goes to NO, i.e., when there is no uncompared comparison target candidate sequence, whether or not there is an uncompared comparison source candidate sequence is checked further (A47). When the flow goes to YES, i.e., when there is an uncompared comparison source candidate sequence, the flow goes back to the step A41 and the same steps are performed subsequently. When the flow goes to NO, i.e., when there is no uncompared comparison source candidate sequence, the process is terminated. In the case where a certain sequence set as a comparison source candidate sequence has already been compared with another sequence set as a comparison target candidate sequence, the comparison between the former sequence as the comparison target candidate sequence and the latter sequence as the comparison source candidate sequence may be omitted, and the result of the comparison may be used.

As described above, by setting the comparison source candidate sequence and the comparison target candidate sequence sequentially from the respective candidate sequences in the candidate sequence group and determining the similarity between the sequences, a similar sequence group including the comparison source candidate sequences and the comparison target candidate sequences similar thereto can be selected.

In the similar information selection device 20 of the present embodiment, the input unit 11 may be connected electrically to the similarity degree calculation unit 131; the similarity degree calculation unit 131 may be connected electrically to the candidate sequence selection unit 132; and the candidate sequence selection unit 132 may be connected electrically to the similar sequence selection unit 133. The similar information selection device 20 may include the respective storage sections, or may not include the respective storage sections, for example. In this case, the similarity degree calculation unit 131 may calculate the similarity degree for each sequence inputted by the input unit 11, the candidate sequence selection unit 132 may select a candidate sequence group using the thus-calculated similarity degrees, and further, the similar sequence selection unit 133 may select a similar sequence group from the selected candidate sequence group, for example.

### [Embodiment 3]

Embodiment 3 relates to the similar information selection device and the similar information selection method of the present invention, similarly to Embodiment 2. The present embodiment is directed to an example where the multiplicity is used in the selection of a similar sequence group in Embodiment 2. Unless otherwise stated, the descriptions in Embodiment 1 and 2 also apply to the present embodiment.

According the present embodiment, a similar sequence group can be selected easily by using the similarity degrees between sequences.

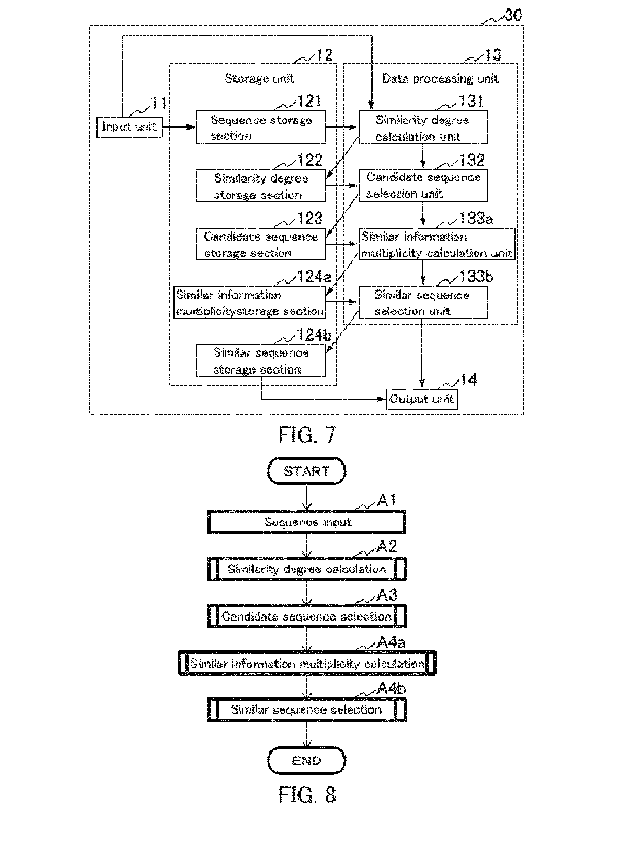

FIG. 7 shows an example of the similar information selection device of the present embodiment. In FIG. 7, components identical to those in the similar information selection device 20 of FIG. 4 are given the same reference numerals. As shown in FIG. 7, the similar information selection device 30 includes: a similar information multiplicity storage section 124a and a similar sequence storage section 124b; and a similar information multiplicity calculation unit 133a and a similar sequence selection unit 133b. The similar information multiplicity calculation unit 133a and the similar sequence selection unit 133b may be incorporated in a data processing unit 13, which is hardware, as shown in FIG. 7, or alternatively, they may be software or hardware with the software installed therein, for example. The similar information multiplicity storage section 124a and the similar sequence storage section 124b may be incorporated in the storage unit 12, which is hardware, as shown in FIG. 7, for example.

The candidate sequence storage section 123 is connected electrically to the similar information multiplicity calculation unit 133a. The similar information multiplicity storage section 124a is connected electrically to the similar information multiplicity calculation unit 133a and the similar sequence selection unit 133b. The similar sequence storage section 124b is connected electrically to the similar sequence selection unit 133b and the output unit 14. The candidate sequence selection unit 132 may be connected electrically to the similar information multiplicity calculation unit 133a. The similar information multiplicity calculation unit 133a may be connected electrically to the similar sequence selection unit 133b. The similar sequence selection unit 133b may be connected electrically to the output unit 14.

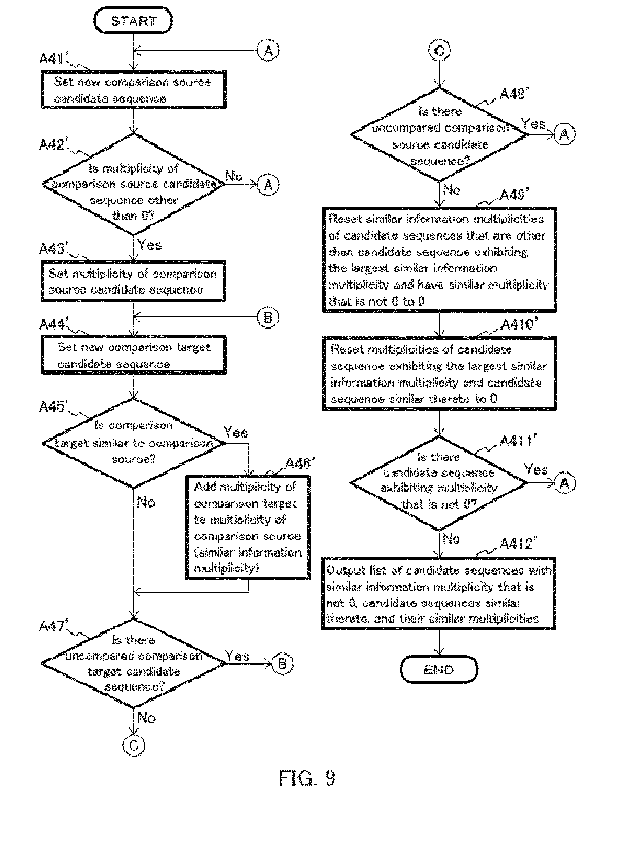

Next, the similar information selection method of the present embodiment will be described with reference to the flowcharts of FIGs. 8 and 9. The similar information selection method of the present embodiment includes the step A1 (sequence input), the step A2 (similarity degree calculation), the step A3 (candidate sequence selection), and the step A4 (similar sequence selection). In the present embodiment, the step A4 includes the step A4a (similar information multiplicity calculation) and the step A4b (similar sequence selection on the basis of the result of the similar information multiplicity calculation). In FIGs. 8 and 9, steps identical to those in FIGs. 5 and 6 are given the same reference numerals.

The steps A1, A2, and A3 can be performed in the same manner as in Embodiment 2. In the present embodiment, the sequence information item to be inputted include, for example, in addition to the order in which components constituting each sequence is aligned, the multiplicity of each sequence.

### (A4) Similar sequence selection

From the candidate sequence group selected in the step A3, a new comparison source candidate sequence is set (A41'), and whether or not the multiplicity of the new comparison source candidate sequence is 0 is determined (A42'). When the flow goes to NO, i.e., when the multiplicity is 0 (the initial multiplicity is 0 or the reset multiplicity is 0), a new comparison source candidate sequence is set again (A41'). On the other hand, when the flow goes to YES, i.e., when the multiplicity is not 0 (the initial multiplicity ≥ 1), the multiplicity of the comparison source candidate sequence is set (A43'). Then, a new comparison target candidate sequence is set (A44'), and whether or not the comparison target candidate sequence is similar to the comparison source candidate sequence is determined (A45'). When the flow goes to YES, i.e., when the comparison target candidate sequence is similar to the comparison source candidate sequence, the sum of the similarity degree of the comparison source candidate sequence and the similarity degree of the comparison target candidate sequence is determined, and the thus-determined sum is set to the similar information multiplicity (A46'). This similar information multiplicity is referred to as the similar information multiplicity of the comparison source candidate sequence. On the other hand, when the flow goes to NO, i.e., when the comparison target candidate sequence is not similar to the comparison source candidate sequence, whether or not there is a comparison target candidate sequence that has not yet been compared with the comparison source candidate sequence is checked (A47'). When the flow goes to YES, i.e., when there is an uncompared comparison target candidate sequence, the flow goes back to the step A44' and the same steps are performed subsequently. Then, when the flow goes to NO, i.e., when there is no uncompared comparison target candidate sequence, whether or not there is an uncompared comparison source candidate sequence is checked further

(A48'). When the flow goes to YES, i.e., when there is an uncompared comparison source candidate sequence, the flow goes back to the step A41' and the same steps are performed subsequently. When the flow goes to NO, i.e., when there is no uncompared comparison source candidate sequence, the similar information multiplicities of candidate sequences that are other than a candidate sequence exhibiting the largest similar information multiplicity and have a similar information multiplicity that is not 0 are reset, i.e., reset to 0 (A49'). Further, the multiplicity of the candidate sequence exhibiting the largest similar information multiplicity and the multiplicities of candidate sequences similar thereto are reset to 0 (A410'). Next, whether or not there is a candidate sequence exhibiting a multiplicity that is not 0 is checked (A411'). When the flow goes to YES, i.e., when there is a candidate sequence exhibiting a multiplicity that is not 0 (the initial multiplicity ≥ 1), this candidate sequence is set as a new comparison source candidate sequence. The flow then goes back to the step A41' and the same steps are performed subsequently. When the flow goes to NO, i.e., when there is no candidate sequence having a multiplicity that is not 0, the candidate sequences having a similar information multiplicity that is not 0 and the candidate sequences similar thereto are set as a similar sequence group, and the list regarding the similar sequence group is outputted (A412'). Examples of the information item to be outputted include respective sequences included in the similar sequence group and the similar information multiplicities.

The step A4 will be described with reference to, as a more specific example, the case where the candidate sequence group includes five kinds of different sequences (Seq1, Seq2, Seq3, Seq4, and Seq5), and the multiplicities (i.e., the number of appearances) of these sequences are {5, 4, 3, 2, and 1}, respectively.

First, the kinds of the candidate sequences and the multiplicities thereof are shown in Table 1 below.

**[Table 1]**

| | | Comparison target | | | | | Multiplicity | Similar information multiplicity |
|---|---|---|---|---|---|---|---|---|
| | | Seq1 | Seq2 | Seq3 | Seq4 | Seq5 | | |
| Comparison source | Seq1 | | | | | | 5 | - |
| | Seq2 | | | | | | 4 | - |
| | Seq3 | | | | | | 3 | - |
| | Seq4 | | | | | | 2 | - |
| | Seq5 | | | | | | 1 | - |

Next, the similarities between the respective pairs of the sequences are determined. In Table 2 below, the sequences that are similar to each other are shaded.

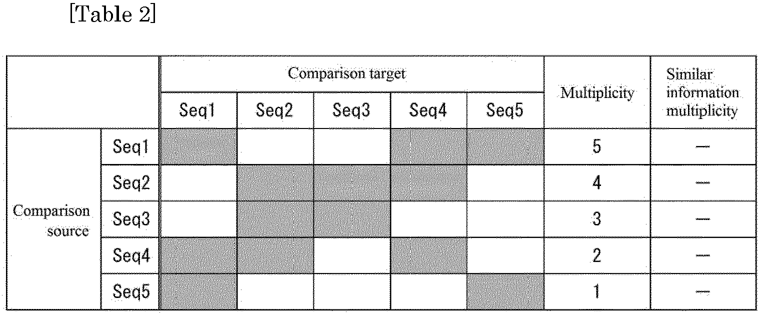

Then, regarding each of the comparison source candidate sequences, the sum of the initial multiplicity of the comparison source candidate sequence and the initial multiplicity of the comparison target candidate sequence(s) similar thereto is determined, and the thus-determined sum is set to the similar information multiplicity of the comparison source candidate sequence. The similar information multiplicities are shown in Table 3 below. Then, among the comparison source candidate sequences, the comparison source candidate sequence exhibiting the largest similar information multiplicity is selected, and the comparison source candidate sequence and the comparison target candidate sequences similar thereto are set as a similar sequence group. In Table 3 below, Seq4 with the largest similar information multiplicity 11 and Seq1 and Seq2 similar to Seq4 belong to the same similar sequence group.

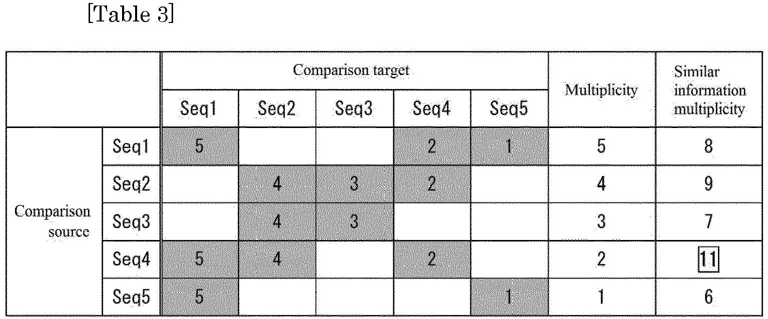

Subsequently, the similar information multiplicities of the comparison source candidate sequences that are other than the comparison source candidate sequence exhibiting the largest similar information multiplicity and have a similar information multiplicity that is not 0 are reset, and the initial multiplicity of the comparison source candidate sequence exhibiting the largest similar information multiplicity and the initial multiplicities of the comparison target candidate sequences similar thereto are reset to 0 (reset multiplicities 0). In Table 4 below, the similar information multiplicities of the sequences other than Seq4 exhibiting the largest similar information multiplicity 11 are reset, and the initial multiplicities of Seq4 and Seq1 and Seq2 similar to Seq4 are reset to 0 (reset multiplicities 0).

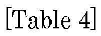

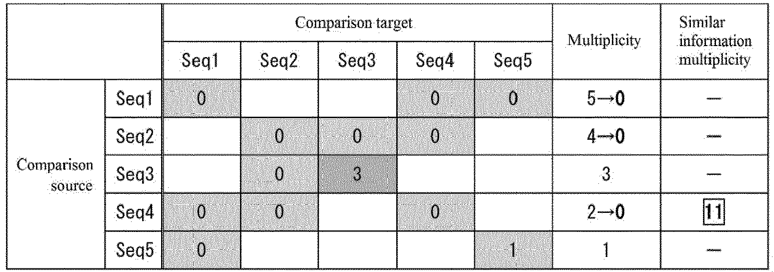

Then, regarding the comparison source candidate sequences exhibiting a multiplicity other than 0 (initial multiplicity ≥1), calculation of the similar information multiplicity and selection of the similar candidate group on the basis of the largest similar information multiplicity are performed in the same manner as in the above. The selection of the similar candidate group preferably is repeated until the initial multiplicities of all the candidate sequences are reset to 0. In Table 5 below, among the candidate sequences having a multiplicity that is not 0, Seq3 exhibiting the largest similar information multiplicity 3 is set as a similar sequence group.

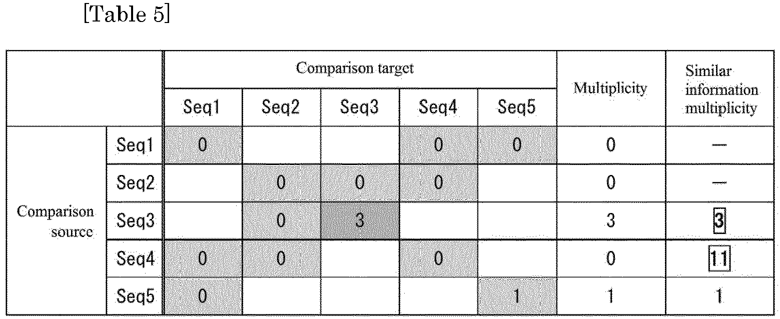

As to the similarity between sequences, it can be said that setting one of the sequences as a comparison source candidate sequence and the other sequence as a comparison target candidate sequence is substantially the same as setting one of the sequences as a comparison target candidate sequence and the other sequence as a comparison source candidate sequence. Thus, from the viewpoint of further accelerating the similar sequence group selection, it is preferable to exclude, as the combination of a comparison source candidate sequence and a comparison target candidate sequence, combinations that have already been made, for example. In this case, for example, as shown in Table 6 below, the number of combinations of different sequences can be reduced by half (the number of the cells is reduced by half).

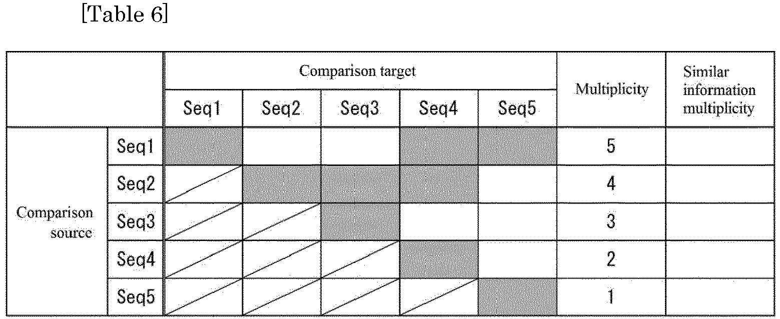

By repeating these processes, it is possible to sort a group of candidate sequences to a group of similar sequences.

### (Device for determining enrichment of desired similar sequence group)

As described above, the enrichment determination device of the present invention is a determination device for determining enrichment of a desired similar sequence information group, including the following units (X) and (Y):
(X) a unit that performs the step of selecting, from a sequence information group including sequence information pieces, a desired sequence information piece and a sequence information piece similar thereto as a desired similar sequence information group; and
(Y) a unit that performs the step of determining enrichment of the similar sequence information group from the sum of the multiplicities of the desired sequence information piece and the sequence information piece similar thereto in the similar sequence information group. In the determination device, the unit (X) is the similar information selection device according to the present invention.

In the determination device of the present invention, the unit (X) is not limited as long as it is the similar information selection device of the present invention, and the descriptions as to the similar information selection device of the present invention also apply to the unit (X).

In the enrichment determination device of the present invention, it is preferable that the unit (X) performs the step of selecting a similar sequence information group that serves as a comparison source and a similar sequence information group that serves as a comparison target, and the unit (Y) is a unit that performs the following steps (Y1) and (Y2):
(Y1) the step of comparing the sum of the multiplicities of a desired sequence information piece and a sequence information piece similar thereto in the comparison source similar sequence information group with the sum of the multiplicities of a desired sequence information piece and a sequence information piece similar thereto in the comparison target similar sequence information group; and
(Y2) the step of determining that the comparison source similar sequence information group is enriched more highly than the comparison target sequence information group, when the sum of the multiplicities in the comparison source similar sequence information group is greater than the sum of the multiplicities in the comparison target similar sequence information group.

In the present invention, the determination of enrichment may be performed by comparing the difference in the degree of enrichment between different sequence information pieces included in the same sequence information group, for example. In this case, for example, the comparison source similar sequence information group and the comparison target similar sequence information group are selected from the same sequence group, and the desired sequence information piece in the comparison source similar sequence information group is different from the desired sequence information piece in the comparison target similar sequence information group. With this configuration, for example, it becomes possible to select, from the same sequence information group, a sequence information piece that is relatively highly enriched and sequence information pieces similar thereto. As a specific example, for example, in aptamer preparation, from a plurality of similar sequence information groups included in a library in a specific round, it is possible to select a relatively highly enriched similar sequence information group, i.e., a highly enriched aptamer similar sequence group.

Also, the determination of enrichment may be performed by, for example, comparing the difference in the degree of enrichment between the same sequence information pieces included in different sequence information groups. In this case, for example, the comparison source similar sequence information group and the comparison target similar sequence information group are selected from different sequence groups, and the desired sequence information piece in the comparison source similar sequence information group is the same as the desired sequence information piece in the comparison target similar sequence information group. With this configuration, for example, from similar sequence information groups including a specific sequence information piece, it is possible to select a relatively highly enriched sequence information group. As a specific example, for example, in aptamer preparation, among libraries of the respective rounds, it is possible to select a library in which a specific aptamer similar sequence group is relatively highly enriched.

The enrichment determination method of the present invention is a determination method for determining enrichment of a similar sequence information group, including the following steps (X) and (Y). Unless otherwise stated, the descriptions as to the enrichment determination device of the present invention also apply to the enrichment determination method of the present invention. The steps (X) and (Y) are:
(X) the step of selecting, from a sequence information group including sequence information pieces, a desired sequence information piece and a sequence information piece similar thereto as a similar sequence information group to be subjected to determination; and
(Y) the step of determining enrichment of the similar sequence information group from the sum of the multiplicities of the desired sequence information piece and the sequence information piece similar thereto in the similar sequence information group. In the determination method, the step (X) is the similar information selection method according to the present invention.

In the enrichment determination method of the present invention, it is preferable that the step (X) is the step of selecting a similar sequence information group that serves as a comparison source and a similar sequence information group that serves as a comparison target, and the step (Y) includes the following steps (Y1) and (Y2):
(Y1) the step of comparing the sum of the multiplicities of a desired sequence information piece and a sequence information piece similar thereto in the comparison source similar sequence information group with the sum of the multiplicities of a desired sequence information piece and a sequence information piece similar thereto in the comparison target similar sequence information group; and
(Y2) the step of determining that the comparison source similar sequence information group is enriched more highly than the comparison target sequence information group, when the sum of the multiplicities in the comparison source similar sequence information group is greater than the sum of the multiplicities in the comparison target similar sequence information group.

In the enrichment determination method of the present invention, the comparison source similar sequence information group and the comparison target similar sequence information group may be similar sequence information groups selected from the same sequence group, and the desired sequence information piece in the comparison source similar sequence information group may be different from the desired sequence information piece in the comparison target similar sequence information group.

In the enrichment determination method of the present invention, the comparison source similar sequence information group and the comparison target similar sequence information group may be similar sequence information groups selected from different sequence groups, and the desired sequence information piece in the comparison source similar sequence information group may be the same as the desired sequence information piece in the comparison target similar sequence information group.

The use of the present invention is not particularly limited. Preferably, the present invention is applied to the determination of enrichment in aptamer preparation, for example. According to the present invention, as described above, it is possible to compare the degree of enrichment between different aptamer similar sequence information groups in the same library or the degree of enrichment between the same aptamer similar sequence information groups in different libraries, for example. Examples

Hereinafter, an example of the present invention will be described. It is to be noted, however, that the present invention is by no means limited by the following example.

### [Example 1]

In the present example, the similar information selection method of the present invention was used to perform sorting to similar sequence groups in a library in which a low molecular weight compound was a target substance.

As a sequence group, a nucleic acid sequence group including 85,800 nucleic acid sequences with a base length of 40-mer was used. The conditions for a virtual sequence group, the allowable number of mismatch bases, and the allowable condition are shown in Table 7 below.

**[Table 7]**

| | Virtual sequence | | Allowable number of mismatches (M) | Allowable condition (NxM) | Calculation time (hour) |
|---|---|---|---|---|---|
| | Base length (N) | Number of sequences | | | |
| Comp. Ex. | - | - | - | - | 8 3 |
| Ex. | 1 | 4 | 5 | 5 | 17 |
| | 2 | 4² | 5 | 10 | 9 |
| | 3 | 43 | 5 | 15 | 1 |
| | 4 | 4⁴ | 5 | 20 | 2 |

In the example, selection of a candidate sequence group and selection of a similar sequence group were carried out under the above conditions, with the number of cells being reduced by half as shown in Table 6 above. Time required for each calculation also is shown in Table 7. In a comparative example, selection of a similar sequence group was carried out by determining the similarities between all the nucleic acid sequences in the sequence group through alignment of these nucleic acid sequences. As a result, according to the example, the similar sequence group could be selected in a markedly shorter calculation time as compared with the comparative example.

While the present invention has been described above with reference to illustrative embodiments, the present invention is by no means limited thereto. Various changes and modifications that may become apparent to those skilled in the art may be made in the configuration and specifics of the present invention without departing from the scope of the present invention.

This application claims priority from Japanese Patent Application No. 2013-027851 filed on February 15, 2013. The entire disclosure of this Japanese patent application is incorporated herein by reference.

### Industrial Applicability

According to the present invention, in order to determine the similarities between sequence information pieces, first, a candidate sequence group for determination of similarity is selected. Thus, for example, unlike conventional methods in which the similarities between all the sequence information pieces are checked, the determination of similarity can be carried out easily and efficiently. Thus, the present invention also can reduce labor, time, and cost for determination of the enrichment of aptamers etc., for example. Explanation of reference numerals

- 10:: candidate selection device
- 20, 30:: similar information selection device
- 11:: input unit
- 12:: storage unit
- 121:: sequence storage section
- 122:: similarity degree storage section
- 123:: candidate sequence storage section
- 124:: similar sequence storage section
- 124a:: similar information multiplicity storage section
- 124b:: similar sequence storage section
- 13:: data processing unit
- 131:: similarity degree calculation unit
- 132:: candidate sequence selection unit
- 133:: similar sequence selection unit
- 133a:: similar information multiplicity calculation unit
- 133b:: similar sequence selection unit
- 14:: output unit

## Claims

1. A candidate selection device for selecting, from a sequence information group comprising sequence information pieces, a candidate sequence information group comprising candidate sequence information pieces that serve as candidates for determination of similarity between the sequence information pieces, the candidate selection device comprising the following units (a), (b), (c), and (d):
(a) a unit that performs the step of counting the frequency of each virtual sequence information piece included in a virtual sequence information group in each sequence information piece in the sequence information group;
(b) a unit that performs the step of selecting, from the sequence information group, a sequence information piece that serves as a comparison source and a sequence information piece that serves as a comparison target;
(c) a unit that performs the step of calculating the difference between the frequency of each virtual sequence information piece in the comparison source sequence information piece and the frequency of each virtual sequence information piece in the comparison target sequence information piece as the similarity degree of the comparison target sequence information piece with respect to the comparison source sequence information piece; and
(d) a unit that performs the step of selecting, when the similarity degree of the comparison target sequence information piece with respect to the comparison source sequence information piece satisfies an allowable similarity degree condition set for the virtual sequence information group, the comparison source sequence information piece and the comparison target sequence information piece as the candidate sequence information group for determination of similarity between the sequence information pieces.

2. The candidate selection device according to claim 1, wherein the virtual sequence information group comprises virtual sequence information pieces constituted by the same components as components constituting the sequence information pieces.

3. The candidate selection device according to claim 1 or 2, wherein the unit (c) is a unit that performs the following steps (c1) and (c2):
(c1) the step of determining, regarding each of the virtual sequence information pieces, the difference between the frequency thereof in the comparison source sequence information piece and the frequency thereof in the comparison target sequence information piece; and
(c2) the step of calculating, as the similarity degree of the comparison target sequence information piece with respect to the comparison source sequence information piece, the absolute value of the sum of positive differences only or the sum of negative differences only among the differences in frequency of the respective virtual sequence information pieces.

4. The candidate selection device according to any one of claims 1 to 3, wherein the allowable similarity degree condition is a condition set based on the allowable number of mismatches when two sequence information pieces are contrasted with each other.

5. The candidate selection device according to any one of claims 1 to 4, wherein the sequence information pieces are base sequences, and components constituting the sequence information pieces are bases A, G, C, T, and U.

6. The candidate selection device according to claim 5, wherein the virtual sequence information pieces have a base length of 1- to 10-mer.

7. The candidate selection device according to claim 5 or 6, wherein the virtual sequence information pieces in the virtual sequence information group all have the same base length.

8. The candidate selection device according to any one of claims 3 to 7, wherein the allowable similarity degree condition is a condition set based on the allowable number of mismatch bases when two sequence information pieces are contrasted with each other.

9. The candidate selection device according to any one of claims 5 to 8, wherein the allowable similarity degree condition is a value obtained by multiplying the allowable number (M) of mismatch bases when two sequence information pieces are contrasted with each other by the base length (N) of the virtual sequence information piece.

10. The candidate selection device according to any one of claims 1 to 9, further comprising the following unit (e):
(e) a unit that repeats the respective steps performed by the units (b), (c), and (d).

11. The candidate selection device according to claim 10, wherein the unit (b) selects, every time the steps are performed, a different sequence information piece from the sequence information group as the comparison source sequence information piece.

12. A similar information selection device for selecting, from a sequence information group comprising sequence information pieces, a similar sequence information group comprising similar sequence information pieces that are similar to each other, the similar information selection device comprising the following units (A) and (B):
(A) a unit that performs the step of selecting, from the sequence information group, a candidate sequence information group comprising candidate sequence information pieces that serve as candidates for determination of similarity between the sequence information pieces; and
(B) a unit that performs the step of contrasting the respective candidate sequence information pieces in the candidate sequence information group with each other and selecting the same and similar sequence information pieces as a similar sequence information group (G3),
wherein the unit (A) is the candidate selection device according to any one of claims 1 to 11.

13. The similar information selection device according to claim 12, wherein the unit (B) is a unit that performs the following steps (B1), (B2), (B3), (B4), and (B5):
(B1) the step of selecting, from the candidate sequence information group, a candidate sequence information piece that serves as a comparison source and a candidate sequence information piece that serves as a comparison target;
(B2) the step of determining whether the comparison target candidate sequence information piece is similar to the comparison source candidate sequence information piece;
(B3) the step of calculating the sum of the multiplicities of the comparison source candidate sequence information piece and the comparison target candidate sequence information piece similar thereto, and setting the calculated sum to the similar information multiplicity of the comparison source candidate sequence information piece;
(B4) the step of selecting, from the candidate sequence information group, a different candidate sequence information piece as a new candidate sequence information piece that serves as a comparison source, and repeating the steps (B1), (B2) and (B3); and
(B5) the step of selecting, among the candidate sequence information pieces, a candidate sequence information piece exhibiting the largest similar information multiplicity and a candidate sequence information piece similar thereto as a similar sequence information group (G3).

14. The similar information selection device according to claim 13, wherein the unit (B) is a unit that further performs the following steps (B6), (B7), and (B8):
(B6) the step of resetting, among the candidate sequence information pieces, the multiplicity of the candidate sequence information piece exhibiting the largest similar information multiplicity and the multiplicity of the candidate sequence information piece similar thereto to 0;
(B7) the step of recalculating the similar information multiplicities of other candidate sequence information pieces exhibiting a multiplicity other than 0; and
(B8) the step of reselecting, among the other candidate sequence information pieces, a candidate sequence information piece exhibiting the largest similar information multiplicity and a candidate sequence information piece similar thereto as a similar sequence information group.

15. The similar information selection device according to claim 14, wherein the unit (B) further performs the following step (B9):
(B9) the step of resetting, among the other candidate sequence information pieces, the multiplicity of the candidate sequence information piece exhibiting the largest similar information multiplicity and the multiplicity of the candidate sequence information piece similar thereto to 0 and repeating the steps (B7) and (B8).

16. The similar information selection device according to any one of claims 13 to 15, wherein the unit (B) excludes, as a combination of the comparison source candidate sequence information piece and the comparison target candidate sequence information piece in the step (B1), a combination that has already been made.

17. A determination device for determining enrichment of a desired similar sequence information group, the determination device comprising the following units (X) and (Y):
(X) a unit that performs the step of selecting, from a sequence information group comprising sequence information pieces, a desired sequence information piece and a sequence information piece similar thereto as a desired similar sequence information group; and
(Y) a unit that performs the step of determining enrichment of the similar sequence information group from the sum of the multiplicities of the desired sequence information piece and the sequence information piece similar thereto in the similar sequence information group,
wherein the unit (X) is the similar information selection device according to any one of claims 12 to 16.

18. The determination device according to claim 17, wherein
the unit (X) performs the step of selecting a similar sequence information group that serves as a comparison source and a similar sequence information group that serves as a comparison target, and
the unit (Y) is a unit that performs the following steps (Y1) and (Y2):
(Y1) the step of comparing the sum of the multiplicities of a desired sequence information piece and a sequence information piece similar thereto in the comparison source similar sequence information group with the sum of the multiplicities of a desired sequence information piece and a sequence information piece similar thereto in the comparison target similar sequence information group; and
(Y2) the step of determining that the comparison source similar sequence information group is enriched more highly than the comparison target sequence information group, when the sum of the multiplicities in the comparison source similar sequence information group is greater than the sum of the multiplicities in the comparison target similar sequence information group.

19. The determination device according to claim 18, wherein
the comparison source similar sequence information group and the comparison target similar sequence information group are selected from the same sequence group, and
the desired sequence information piece in the comparison source similar sequence information group is different from the desired sequence information piece in the comparison target similar sequence information group.

20. The determination device according to claim 18, wherein
the comparison source similar sequence information group and the comparison target similar sequence information group are selected from different sequence groups, and
the desired sequence information piece in the comparison source similar sequence information group is the same as the desired sequence information piece in the comparison target similar sequence information group.

21. A candidate selection method for selecting, from a sequence information group including sequence information pieces, a candidate sequence information group including candidate sequence information pieces that serve as candidates for determination of similarity between the sequence information pieces, the candidate selection method comprising the following steps (a), (b), (c), and (d):
(a) the step of counting the frequency of each virtual sequence information piece included in a virtual sequence information group in each sequence information piece in the sequence information group;
(b) the step of selecting, from the sequence information group, a sequence information piece that serves as a comparison source and a sequence information piece that serves as a comparison target;
(c) the step of calculating the difference between the frequency of each virtual sequence information piece in the comparison source sequence information piece and the frequency of each virtual sequence information piece in the comparison target sequence information piece as the similarity degree of the comparison target sequence information piece with respect to the comparison source sequence information piece; and
(d) the step of selecting, when the similarity degree of the comparison target sequence information piece with respect to the comparison source sequence information piece satisfies an allowable similarity degree condition set for the virtual sequence information group, the comparison source sequence information piece and the comparison target sequence information piece as the candidate sequence information group for determination of similarity between the sequence information pieces.

22. The candidate selection method according to claim 21, wherein the virtual sequence information group comprises virtual sequence information pieces constituted by the same components as components constituting the sequence information pieces.

23. The candidate selection method according to claim 21 or 22, wherein the step (c) comprises the following steps (c1) and (c2):
(c1) the step of determining, regarding each of the virtual sequence information pieces,
the difference between the frequency thereof in the comparison source sequence information piece and the frequency thereof in the comparison target sequence information piece; and
(c2) the step of calculating, as the similarity degree of the comparison target sequence information piece with respect to the comparison source sequence information piece, the absolute value of the sum of positive differences only or the sum of negative differences only among the differences in frequency of the respective virtual sequence information pieces.

24. The candidate selection method according to any one of claims 21 to 23, wherein the allowable similarity degree condition is a condition set based on the allowable number of mismatches when two sequence information pieces are contrasted with each other.

25. The candidate selection method according to any one of claims 21 to 24, wherein the sequence information pieces are base sequences, and components constituting the sequence information pieces are bases A, G, C, T, and U.

26. The candidate selection method according to claim 25, wherein the virtual sequence information pieces have a base length of 1- to 10-mer.

27. The candidate selection method according to claim 25 or 26, wherein the virtual sequence information pieces in the virtual sequence information group all have the same base length.

28. The candidate selection method according to any one of claims 23 to 27, wherein the allowable similarity degree condition is a condition set based on the allowable number of mismatch bases when two sequence information pieces are contrasted with each other.

29. The candidate selection method according to any one of claims 25 to 28, wherein the allowable similarity degree condition is a value obtained by multiplying the allowable number (M) of mismatch bases when two sequence information pieces are contrasted with each other by the base length (N) of the virtual sequence information piece.

30. The candidate selection method according to any one of claims 21 to 29, further comprising the following step (e):
(e) the step of repeating the steps (b), (c), and (d).

31. The candidate selection method according to claim 30, wherein the step (b) is such that, every time the steps are performed, a different sequence information piece is selected from the sequence information group as the comparison source sequence information piece.

32. A similar information selection method for selecting, from a sequence information group comprising sequence information pieces, a similar sequence information group comprising similar sequence information pieces that are similar to each other, the similar information selection method comprising the following steps (A) and (B):
(A) the step of selecting, from the sequence information group, a candidate sequence information group comprising candidate sequence information pieces that serve as candidates for determination of similarity between the sequence information pieces; and
(B) the step of contrasting the respective candidate sequence information pieces in the candidate sequence information group with each other and selecting the same and similar sequence information pieces as a similar sequence information group (G3),
wherein the step (A) comprises the candidate selection method according to any one of claims 21 to 31.

33. The similar information selection method according to claim 32, wherein the step (B) comprises the following steps (B1), (B2), (B3), (B4), and (B5):
(B1) the step of selecting, from the candidate sequence information group, a candidate sequence information piece that serves as a comparison source and a candidate sequence information piece that serves as a comparison target;
(B2) the step of determining whether the comparison target candidate sequence information piece is similar to the comparison source candidate sequence information piece;
(B3) the step of calculating the sum of the multiplicities of the comparison source candidate sequence information piece and the comparison target candidate sequence information piece similar thereto, and setting the calculated sum to the similar information multiplicity of the comparison source candidate sequence information piece;
(B4) the step of selecting, from the candidate sequence information group, a different candidate sequence information piece as a new candidate sequence information piece that serves as a comparison source, and repeating the steps (B1), (B2) and (B3); and
(B5) the step of selecting, among the candidate sequence information pieces, a candidate sequence information piece exhibiting the largest similar information multiplicity and a candidate sequence information piece similar thereto as a similar sequence information group (G3).

34. The similar information selection method according to claim 33, wherein the step (B) further comprises the following steps (B6), (B7) and (B8):
(B6) the step of resetting, among the candidate sequence information pieces, the multiplicity of the candidate sequence information piece exhibiting the largest similar information multiplicity and the multiplicity of the candidate sequence information piece similar thereto to 0;
(B7) the step of recalculating the similar information multiplicities of other candidate sequence information pieces exhibiting a multiplicity other than 0; and
(B8) the step of reselecting, among the other candidate sequence information pieces, a candidate sequence information piece exhibiting the largest similar information multiplicity and a candidate sequence information piece similar thereto as a similar sequence information group.

35. The similar information selection method according to claim 34, wherein the step (B) further comprises the following step (B9):
(B9) the step of resetting, among the other candidate sequence information pieces, the multiplicity of the candidate sequence information piece exhibiting the largest similar information multiplicity and the multiplicity of the candidate sequence information piece similar thereto to 0 and repeating the steps (B7) and (B8).

36. The similar information selection method according to any one of claims 33 to 35, wherein the step (B) comprises excluding, as a combination of the comparison source candidate sequence information piece and the comparison target candidate sequence information piece in the step (B1), a combination that has already been made.

37. A determination method for determining enrichment of a similar sequence information group, the determination method comprising the following steps (X) and (Y):
(X) the step of selecting, from a sequence information group comprising sequence information pieces, a desired sequence information piece and a sequence information piece similar thereto as a similar sequence information group to be subjected to determination; and
(Y) the step of determining enrichment of the similar sequence information group from the sum of the multiplicities of the desired sequence information piece and the sequence information piece similar thereto in the similar sequence information group,
wherein the step (X) comprises the similar information selection method according to any one of claims 32 to 36.

38. The determination method according to claim 37, wherein
the step (X) is the step of selecting a similar sequence information group that serves as a comparison source and a similar sequence information group that serves as a comparison target, and
the step (Y) comprises the following steps (Y1) and (Y2):
(Y1) the step of comparing the sum of the multiplicities of a desired sequence information piece and a sequence information piece similar thereto in the comparison source similar sequence information group with the sum of the multiplicities of a desired sequence information piece and a sequence information piece similar thereto in the comparison target similar sequence information group; and
(Y2) the step of determining that the comparison source similar sequence information group is enriched more highly than the comparison target sequence information group,
when the sum of the multiplicities in the comparison source similar sequence information group is greater than the sum of the multiplicities in the comparison target similar sequence information group.

39. The determination method according to claim 38, wherein
the comparison source similar sequence information group and the comparison target similar sequence information group are selected from the same sequence group, and
the desired sequence information piece in the comparison source similar sequence information group is different from the desired sequence information piece in the comparison target similar sequence information group.

40. The determination method according to claim 38, wherein
the comparison source similar sequence information group and the comparison target similar sequence information group are selected from different sequence groups, and
the desired sequence information piece in the comparison source similar sequence information group is the same as the desired sequence information piece in the comparison target similar sequence information group.

41. A program that can execute the candidate selection method according to any one of claims 21 to 31 on a computer.

42. A program that can execute the similar information selection method according to any one of claims 32 to 36 on a computer.

43. A program that can execute the determination method according to any one of claims 37 to 40 on a computer.

44. A recording medium having recorded thereon the program according to any one of claims 41 to 43.
